# FASCICULE DE BREVET EUROPEEN

(11) **EP 1 727 543 B1**
(45) Date de publication et mention de la délivrance du brevet: **15.08.2012**
(21) Numéro de dépôt: 05717501.0
(22) Date de dépôt: 27.01.2005
(51) Int. Cl.: A61K 31/4375, A61K 9/16, A61K 9/20, A61K 9/48, A61P 25/24

(54) **UTILISATION DU 14,15-DIHYDRO 20,21-DINOREBURNAMENIN14-OL POUR TRAITER ET/OU PREVENIR LES DEPRESSIONS MAJEURES**
VERWENDUNG VON 14,15-DIHYDRO-20,21-DINOREBURNAMENIN-14-OL ZUR BEHANDLUNG UND PRÄVENTION VON SCHWERER DEPRESSION
USE OF 14,15-DIHYDRO-20,21-DINOREBURNAMENIN-14-OL FOR THE TREATMENT AND/OR PREVENTION OF SERIOUS DEPRESSION

(30) Priorité: 30.01.2004 FR 0400905; 08.04.2004 FR 0403694
(43) Date de publication de la demande: 06.12.2006
(73) Titulaire: MARCO POLO PHARMACEUTICALS, 75008 Paris (FR)
(72) Inventeur: PUJOL, Jean-François, F-75007 Paris (FR); WEISSMANN, Dinah, F-75007 Paris (FR)
(74) Mandataire: Faivre Petit, Frédérique
(86) Numéro de dépôt international: PCT/FR2005/000178
(87) Numéro de publication internationale: WO 2005/082365

(56) Documents cités:
- FR-A- 2 590 572
- RU-C- 2 043 353
- US-A- 4 291 038
- US-A- 5 034 396
- US-A- 5 093 337
- US-A- 5 332 748
- US-A1- 2002 045 637

## Description

L'invention a pour objet une nouvelle application thérapeutique du 14,15-dihydro 20,21-dinoréburnaménin14-ol pour le traitement des dépressions majeures chez l'Homme, notamment pour le traitement de patient résistant aux traitements antidépresseurs classiques,

La dépression est l'un des troubles psychologiques les plus fréquents. En France, le taux de dépressifs est de 14,9 % dont près d'un tiers n'est pas pris en charge médicalement. Une femme sur cinq serait touchée.

La prévalence de la dépression déclarée a été multipliée par 6 depuis 1970. Entre 1992 et 1997, le taux de dépressifs a particulièrement augmenté chez les jeunes de 20 à 29 ans (+ 65 %), qui ont été touchés dans le même temps par une hausse de 20 % du chômage.

Le risque de présenter une dépression majeure au cours de la vie varie, selon les études, de 10 à 25 % pour les femmes et de 5 à 12 % pour les hommes.

La dépression majeure constitue l'une des catégories de dépressions répertoriées dans le DSM IV (classification américaine des troubles mentaux), et est caractérisée par les symptômes qu'elle présente. Notamment, la dépression majeure, auparavant nommée dépression mélancolique, est à distinguer des autres groupes cliniques tels que les états dépressifs réactionnels, les dépressions dues à l'épuisement, les dépressions liées au terrain (dépression de l'enfant, de la femme enceinte, de la personne âgée) ou les dépressions saisonnières.

Il est donc particulièrement indispensable de trouver des traitements mieux adaptés à ce type de dépression, d'autant que certains patients ne répondent pas aux antidépresseurs classiques.

Les dérivés de la 20,21-dinoréburnaménine, dont le 14,15-dihydro 20,21-dinorébumaménin14-ol, sont déjà connus pour leurs propriétés vasodilatatrices, notamment cérébrales et pour leur activité dans la régulation de la tyrosine hydroxylase dans le locus coeruleus (Bourde et al., Neurochem. Int., 23 (6), 567-574, 1993). Ils sont utilisés dans les vasculopathies cérébrales et tous les syndromes provoqués par une altération de la circulation cérébrale.

Ces dérivés ainsi que leur première application thérapeutique connue ont été décrits dans la demande de brevet FR 2 381 048, publiée le 15 septembre 1978. Cette demande de brevet a fait l'objet d'une demande de certificat d'addition FR 2 433 528 publiée le 14 mars 1980.

Plus particulièrement, la demande FR 2 381 048 décrit les dérivés de la 20,21-dinoréburnaménine et leur procédé de préparation. Sont également décrites, les propriétés pharmacologiques de ces composés: ces composés sont des oxygénateurs et vasorégulateurs cérébraux de grande valeur, qui entraînent en particulier une augmentation du flux cérébral au niveau de la microcirculation cérébrale.

D'autre part, la demande FR 2 433 528 décrit le procédé de préparation d'un isomère particulier dérivé de la 20,21-dinoréburnaménine, et l'isomère obtenu par ce procédé.

La demande WO 89/04830, publiée le 1er juin 1989, décrit de nouveaux dérivés substitués de la 20,21-dinoréburnaménine, leur procédé de préparation et leur application comme médicament notamment comme antidépresseur.

La dépression est un état psychique pathologique associant une modification pénible de l'humeur et un ralentissement de l'activité intellectuelle et motrice. C'est un état morbide, plus ou moins durable, caractérisé par la tristesse et une diminution du tonus de l'énergie.

Les principaux symptômes permettant de diagnostiquer une dépression chez une personne sont les suivants :
- humeur dépressive,
- diminution marquée de l'intérêt ou du plaisir,
- troubles de l'alimentation,
- troubles du sommeil,
- agitation ou ralentissement psychomoteur,
- fatigue ou perte d'énergie,
- autodévalorisation ou sentiment de culpabilité excessive,
- diminution de l'aptitude à penser ou à se concentrer ou indécision,
- pensées morbides (dans 60 % des cas),
- pensées suicidaires (dans 15 % des cas).

Parmi les facteurs causals de la dépression, on peut citer :

### 1/ Le facteur héréditaire

Les personnes dont les parents proches souffrent ou ont souffert d'une dépression sont plus susceptibles d'en être atteintes. Elles ont 15 % de risque de développer une dépression alors que les personnes dont les parents proches ne sont pas dépressifs ont seulement 2 à 3 % de risque d'en développer une

### 2/ Le facteur biochimique

Les recherches actuelles sur la dépression portent sur les neurotransmetteurs. On a ainsi pu remarquer qu'une déficience ou un déséquilibre de la sérotonine entraînait une perte de sommeil ainsi qu'une diminution de l'appétit. Mais aussi qu'une baisse de la noradrénaline influe sur la perte d'énergie, le manque de plaisir.

### 3/ Les facteurs liés à l'environnement

Les enfants ayant vécu la perte d'un être cher comme leurs parents, sont plus sujets à développer des dépressions plus tard dans leur vie. Des difficultés dans les relations, des problèmes de communication ainsi que des conflits familiaux, professionnels ou autres, peuvent aussi contribuer à la solitude, à l'aliénation et aboutir à la dépression. Les difficultés financières et autres tensions peuvent également avoir un impact important.

Il ne faut pas négliger les facteurs saisonniers : le taux de dépression est plus élevé au cours des mois où l'ensoleillement est le plus faible.

L'art antérieur connaît deux grands types de traitements pour la dépression.

Le traitement médicamenteux avec les antidépresseurs, indiqués dans toutes les formes de dépressions. Ils agissent sur l'équilibre des neurotransmetteurs.

Les antidépresseurs sont efficaces chez 75 % des personnes souffrant de dépression sévère.

Les psychothérapies : elles viennent en aide aux malades mais ne peuvent agir comme seul traitement.

Il existe d'autres formes de traitements comme les thérapies comportementales et cognitives (concerne surtout les dépressions névrotiques), la sismothérapie ou électrochoc (utilisée en dernier recours).

L'évolution de la dépression est très variable et fonction de nombreux paramètres : étiologie, personnalité du sujet, ....

En dehors de tout traitement, il arrive couramment que la dépression dure 6 mois voire plus avec dans certains cas, comme terminaison extrême, le suicide. Jusqu'à 15 % des sujets présentant un trouble dépressif majeur sévère se suicident.

La dépression peut être diagnostiquée par les critères du DSM IV (Diagnostic and Statistical Manual of Mental Disorders, 4th edition, American Psychiatric Association Publisher ; Washington DC) ; le DSM IV est un référentiel diagnostique et statistique des troubles mentaux élaboré par l'American Psychiatry Association.

Selon les critères du DSM IV, la dépression majeure (dénommée encore MDD pour « Major Depressive Disorders ») se distingue notamment des troubles dysthymiques (« dépression mineure ») caractérisés par une dépression chronique mais moins sévère que la dépression majeure et dont l'épisode peut durer au moins deux ans, épisode pouvant conduire à une dépression majeure dans plus d'un tiers des cas.

Selon les critères du DSM IV, la dépression majeure qui est la forme sévère et la plus commune de la dépression et pour laquelle seulement 10 à 25 % des patients recherchent un traitement, est caractérisée par un ou plusieurs épisodes de changement d'humeur ou de perte d'intérêt d'au moins deux semaines accompagnée d'au moins quatre symptômes additionnels de dépression ; ces derniers symptômes peuvent être par exemple un changement de l'appétit, du poids, du sommeil, ou de l'activité psychomotrice ; une diminution de l'énergie, un sentiment de dévalorisation ou de culpabilité, une difficulté à réfléchir, à se concentrer, à prendre des décisions, ou bien des pensées récurrentes de mort, ou l'idéation, des projets, des tentatives de suicide.

Pour pouvoir caractériser un épisode dépressif majeur, un symptôme doit être soit nouvellement présent, soit avoir empiré comparé à l'état de la personne lors d'un épisode antérieur. Les symptômes doivent persister durant la plus grande partie de la journée, quasiment tous les jours, pendant au moins deux semaines consécutives. Cet épisode doit être accompagné d'une détresse significative d'un point de vue clinique ou d'une détérioration du fonctionnement social, occupationnel. Chez certaines personnes ayant des épisodes plus bénins, le fonctionnement peut apparaître normal mais nécessite un effort particulièrement marqué.

Par définition, un épisode dépressif majeur n'est pas dû aux effets physiologiques directs d'un abus de drogue (par exemple, dans un contexte de sevrage/manque lors d'intoxication à l'alcool ou à la cocaïne) ni aux effets secondaires lors de médications ou de traitements (par exemple les stéroïdes), ni à l'exposition à une toxine. De manière similaire, l'épisode n'est pas dû aux effets physiologiques directs d'un état médical de manière générale (par exemple l'hyperthyroïdisme).

Parmi les dépressions majeures, on peut citer les dépressions résistant au traitement par antidépresseurs classiques (dénommées TRD pour « Treatment Resistant Depression »). Il apparaît en effet que 30 à 46 % des patients déprimés montrent une réponse partielle ou pas de réponse aux antidépresseurs (Fava et al., Psychiatric Clin. North Am., 19, 2:179-200, 1996).

Les antidépresseurs classiques actuellement et couramment commercialisés appartiennent aux classes principales suivantes :
- antidépresseurs tricycliques (TCA),
- les inhibiteurs de monoamine oxydase (MAO) (MAOIs),
- les inhibiteurs sélectifs de recapture de la sérotonine (SSRIs),
- les inhibiteurs de recapture de la noradrénaline et de la sérotonine (SNDRIs),
- les antidépresseurs sélectifs de la sérotonine et de la noradrénaline (NASSAs),
- les modulateurs du récepteur à la sérotonine.

Les dépressions résistant au traitement (TRD) représentent une forme plus handicapante et chronique de MDD (Kornstein et al., J. Clin. Psychiatry, 62, suppl. 16:18-25, 2001). Selon le modèle proposé par Thase et al. (J. Clin. Psychiatry, 58, suppl. 13:23-29,1997), les stades de la TRD peuvent être évalués comme suit :
- Stade I : Echec à au moins un essai adéquate d'un antidépresseur d'une classe majeure ;
- Stade II : Echec stade I plus échec à un essai adéquate d'un antidépresseur d'une classe différente de celle utilisée au stade I ;
- Stade III : Echec stade II plus échec à un essai adéquate d'un antidépresseur tricyclique ;
- Stade IV : Echec stade III plus échec à un essai adéquate d'un inhibiteur MAO ; et
- Stade V : Echec stade IV plus échec à un traitement par thérapie par électrochoc bilatéral (ECT).

Le Massachusetts Général Hospital (MGH Boston) a déterminé une méthode pour classer le processus TRD à partir de :
- Item 1 : aucune réponse pour chaque essai à un antidépresseur commercial générant un score global de résistance (1 point par essai) (au moins 6 semaines d'une dose adéquate d'antidépresseur) ;
- Item 2 : optimisation du dosage, optimisation de la durée et augmentation / combinaison de chaque essai (basées sur le MGH ou le questionnaire de réponse au traitement antidépresseur) (0,5 point par essai et par optimisation / stratégie) ; et
- Item 3 : l'ECT augmente le total de 3 points.

On peut noter le fait que la TRD est un événement relativement commun dans la pratique clinique, avec plus de 50 à 60 % de patients ne réalisant pas les réponses adéquates après un traitement antidépresseur.

Parmi ces dépressions majeures, on peut citer également les troubles dépressifs récurrents majeurs (dénommés également MRDD pour « Major Recurrent Depressive Disorders »), troubles qui sont associés à des épisodes hypomaniaques.

La sévérité des ces troubles dépressifs, de la forme légère à sévère, peut être évaluée au moyen d'échelles numériques classiques et validées, telle que l'échelle HAMD (« Hamilton Depression Scale ») ou encore l'échelle MADRS (Montgomery and Asberg Depression Rating Scale) qui sont les plus utilisées. Selon ces échelles, une dépression sera considérée comme sévère si ses symptômes aboutissent à un score supérieur à 26 pour l'échelle HAMD ou à 35 pour l'échelle MADRS.

Les troubles du sommeil touchent une part toujours plus importante de la population. On évalue à au moins 20 % le taux de la population souffrant de troubles du sommeil en Europe, aux Etats-unis ou en Australie. Deux études portant sur de larges échantillons de la population française trouvent un taux de prévalence de 22 %. Un Français sur 6 se plaint de son sommeil (près de 9 millions).

On assiste, avec l'âge, à une aggravation et à une chronicité de la désorganisation du sommeil. 60 à 70 % des consommateurs réguliers d'hypnotiques et d'anxiolytiques ont plus de 40 ans. Reste que la sévérité de l'insomnie chez l'enfant reste mal connue, les enquêtes reposant le plus souvent sur l'appréciation des parents, qui sous-estiment les troubles. Un questionnaire de sommeil adressé à des adolescents de 16 à 19 ans a pu montrer que 14 % d'entre eux avaient des difficultés d'endormissement, 8 % des éveils nocturnes fréquents et 6 % des éveils trop matinaux.

Ainsi, il reste à pouvoir disposer de composés capables de traiter les troubles d'une dépression majeure chez un patient ou de traiter des patients « TRD » atteints de dépression, notamment majeure, qui sont résistants au traitement par les antidépresseurs classiques comme cités ci-avant, et/ou à prévenir le traitement des désordres du cycle veille-sommeil.

Ceci est justement l'objet de l'invention décrite et revendiquée ci-après.

Il a été découvert de manière surprenante que l'utilisation du 14,15-dihydro 20,21-dinoréburnaménin14-ol, dénommé ici BC19 lorsqu'il est sous la forme d'un mélange racémique, permettait de traiter des patients atteints de dépression majeure et/ou de TRD et/ou à prévenir le traitement des désordres du cycle veille-sommeil.

D'autre part, il a été également mis en évidence de manière surprenante que l'utilisation du 14,15-dihydro 20,21-dinoréburnaménin14-ol permettait de rendre sensibles aux traitements antidépresseurs classiques les patients atteints de dépression majeure qui étaient résistants à ces traitements.

L'invention a donc pour objet l'utilisation d'un composé de formule (I) 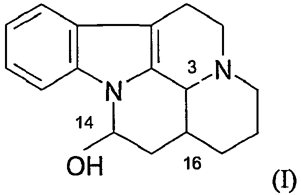 ou l'un de ses sels pharmaceutiquement acceptables, pour la préparation d'une composition pharmaceutique destinée au traitement ou la prévention des dépressions majeures (MDD),

De préférence lesdits désordres du cycle veille-sommeil sont choisis parmi la narcolepsie, l'hypersomnie, et l'état chronique d'hypoéveil.

Sous un autre aspect, l'invention a pour objet l'utilisation d'un composé de formule (I) ou l'un de ses sels pharmaceutiquement acceptables, pour la préparation d'une composition pharmaceutique destinée au traitement ou à la prévention de patients atteints de dépression et résistant partiellement ou totalement aux traitements par les antidépresseurs classiques (patients atteints de TRD), comme les antidépresseurs appartenant à la classe des antidépresseurs tricycliques (TCA), les inhibiteurs de monoamine oxydase (MAOIs), les inhibiteurs sélectifs de recapture de la sérotonine, (SSRIs)les inhibiteurs de recapture de la noradrénaline et de la sérotonine (SNDRIs), les antidépresseurs sélectifs de la sérotonine et de la noradrénaline (NASSAs) ou les modulateurs du récepteur à la sérotonine.

Sous un aspect préféré, l'invention concerne l'utilisation d'un composé de formule (I) ou l'un de ses sels pharmaceutiquement acceptables, pour la préparation d'une composition pharmaceutique destinée au traitement ou à la prévention de patients atteints de dépression majeure et résistant partiellement ou totalement aux traitements par les antidépresseurs classiques (patients atteints de MDD et TRD).

Sous un aspect particulier, l'invention concerne l'utilisation d'un composé de formule (I) ou l'un de ses sels pharmaceutiquement acceptables selon l'invention, caractérisée en ce que la dépression majeure est une dépression de type bipolaire selon la nomenclature du DSM IV, notamment un trouble dépressif récurrent majeur (MRDD).

Sous un aspect particulier, l'invention concerne l'utilisation d'un composé de formule (I) ou l'un de ses sels pharmaceutiquement acceptables selon l'invention, caractérisée en ce que la sévérité de la dépression lorsqu'elle est évaluée par l'échelle HAMD (« Hamilton Depression Scale ») a un score supérieur à 26 ou par l'échelle MADRS (Montgomery and Asberg Depression Rating Scale) a un score supérieur à 35.

Sous encore un autre aspect, l'invention a pour objet l'utilisation d'un composé de formule (I) ou l'un de ses sels pharmaceutiquement acceptables, pour la préparation d'une composition pharmaceutique destinée à rendre sensibles aux traitements antidépresseurs classiques des patients atteints de dépression majeure résistant à ces traitements.

Par sels d'addition pharmaceutiquement acceptables, on peut citer ici comme exemple les sels d'addition avec les acides minéraux ou organiques, notamment les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, ascorbique, les acides alcoylmonosulfoniques, tels que l'acide méthanesulfonique, l'acide éthane sulfonique, l'acide propane sulfonique, les acides alcoyldisulfoniques tels que l'acide méthanedisulfonique, l'acide α,β-éthanedisulfonique, et les acides arylmonosulfoniques, tels que l'acide benzènesulfonique et les acides aryldisulfoniques, ces sels n'étant mentionnés qu'à titre illustratif mais sans constituer une limitation.

Le composé de formule (I) est caractérisé par deux formes énantiomériques 3α et 16α, et est caractérisé pour chacun de ces énantiomères par un couple de diastéréoisomères selon la configuration du carbone 14 :
- le couple (3α, 14α) 14,15-dihydro 20,21-dinoréburnaménin14-ol et (3α, 14β) 14,15-dihydro 20,21-dinoréburnaménin14-ol ; et
- le couple (14α, 16α) 14,15-dihydro 20,21-dinoréburnaménin14-ol et (-) (14β, 16α) 14,15-dihydro 20,21-dinoréburnaménin14-ol.

Les pouvoirs rotatoires ont été mesurés, ce qui a permis de déterminer les signes (+) et (-) attribués à chaque isomère.

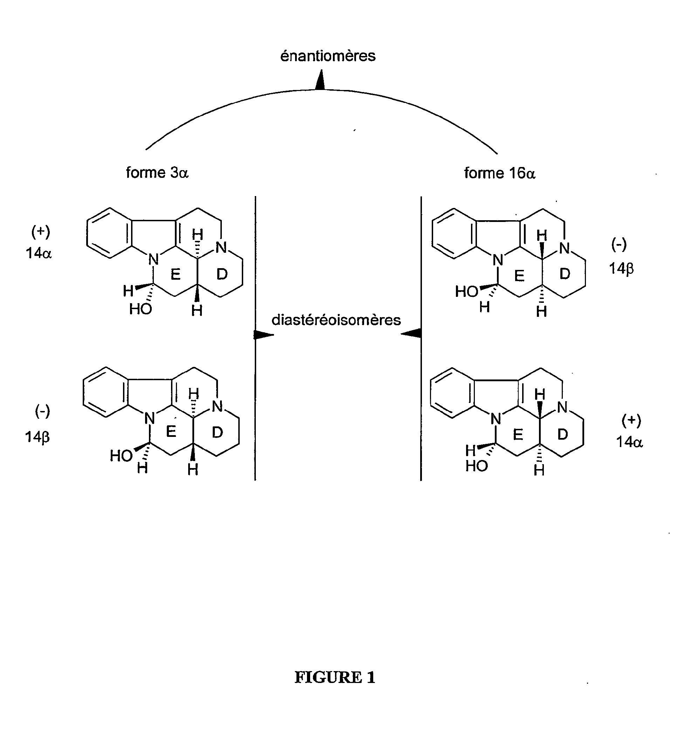

Les différents composés sont décrits dans la figure 1 de l'article publié par Bourde et al. (Neurochem Int., 23:567-574, 1993).

L'invention a donc pour objet selon un aspect particulier l'utilisation d'un composé de formule (I) ou l'un de ses sels pharmaceutiquement acceptables dans laquelle le composé de formule (I) ou l'un de ses sels pharmaceutiquement acceptables est sous la forme d'un mélange racémique ou optiquement actif.

L'invention a en outre pour objet l'utilisation selon la présente invention, caractérisée en ce que le composé de formule (I) ou l'un de ses sels pharmaceutiquement acceptables est choisi parmi les composés de formule (I) suivants :
a) (3α) (±) 14,15-dihydro 20,21-dinoréburnaménin 14-ol ; et
b) (16α) (±) 14,15-dihydro 20,21-dinoréburnaménin 14-ol,
et dans lequel, le mélange des deux diastéréoisomères (+) et (-) présents dans ces composés a) et b) est en proportion équimolaire ou non.

L'invention a aussi pour objet l'utilisation selon la présente invention, caractérisée en ce que le composé de formule (I) ou l'un de ses sels pharmaceutiquement acceptables est choisi parmi les composés de formule (I) suivants :
a) (3α, 14α) 14,15-dihydro 20,21-dinoréburnaménin 14-ol ;
b) (3α, 14β) 14,15-dihydro 20,21-dinoréburnaménin 14-ol ;
c) (14α, 16α) 14,15-dihydro 20,21-dinoréburnaménin 14-ol ; et
d) (14β, 16α) 14,15-dihydro 20,21-dinoréburnaménin 14-ol.

De manière plus particulière, l'invention a donc pour objet l'utilisation d'un composé de formule (I) ou l'un de ses sels pharmaceutiquement acceptables selon la présente invention, pour la préparation d'une composition pharmaceutique administrable par voie orale, par voie intraveineuse, par voie intrapéritonéale, par voie intramusculaire ou par toute autre voie permettant d'obtenir un effet antidépresseur selon la présente invention, ou de rendre sensibles aux traitements antidépresseurs classiques les patients atteints de dépression majeure qui étaient résistants à ces traitements.

Les substances actives des compositions pharmaceutiques selon l'invention peuvent être dans n'importe laquelle des formes galéniques orales habituelles comprenant des comprimés, des capsules et des préparations liquides telles que des élixirs et des suspensions contenant diverses substances masquantes de coloration, de saveur et de stabilisation.

Pour réaliser les formes galéniques orales selon l'invention, la substance active peut être mélangée à divers matériaux conventionnels tels que l'amidon, le carbonate de calcium, le lactose, le sucrose et le phosphate dicalcique pour faciliter le processus d'encapsulation. Le stéarate de magnésium, comme additif, fournit une fonction utile de lubrifiant si nécessaire.

Les substances actives des compositions pharmaceutiques selon l'invention peuvent être dissoutes ou mises en suspension dans un liquide stérile pharmaceutiquement acceptable, tel que l'eau stérile, un solvant organique stérile ou un mélange de ces deux liquides. De préférence, un tel liquide est approprié pour l'injection parentérale.

Lorsque la substance active est suffisamment soluble elle peut être dissoute dans une solution saline normale telle qu'un liquide stérile pharmaceutiquement acceptable ; si elle est insuffisamment soluble, elle peut être dissoute dans les solutions aqueuses d'un dissolvant organique approprié, par exemple de glycol de propylène ou de polyéthylène glycol. Le glycol de propylène aqueux contenant de 10 à 75 % en poids du glycol est généralement approprié. Dans d'autres exemples, d'autres compositions peuvent être obtenues en dispersant la substance active en concentré très fin dans la solution carboxyméthylique aqueuse de cellulose d'amidon ou de sodium, ou dans une huile appropriée, par exemple huile d'arachide.

Des compositions pharmaceutiques liquides telles que les solutions ou les suspensions stériles peuvent être utilisées pour l'injection intramusculaire, intrapéritonéale ou sous-cutanée.

De préférence la composition pharmaceutique est sous la forme de doses unitaires, par exemple comme comprimés ou capsules. Sous une telle forme, la composition est subdivisée dans des doses d'unité contenant des quantités appropriées de la substance active ; les doses unitaires peuvent être les compositions emballées, par exemple des poudres, des fioles ou des ampoules. La quantité de la substance active dans une dose d'unité de composition peut être modifiée ou ajustée de 2 mg ou moins de 50 mg ou plus, selon le besoin particulier et l'activité de la substance active.

La dose orale recommandée de 14,15-dihydro 20,21-dinoréburnaménin14-ol pour l'homme peut être de 20 à 60 mg/jour et cette dose peut être administrée en deux ou trois doses séparées, de préférence lors d'un repas. La plupart des patients mélancoliques résistants répondent à la dose de 20 mg/jour, mais 40 mg, voire 60 mg peuvent être nécessaires.

L'homme de l'art sait également que les voies d'administration des composés selon cette invention peuvent changer de manière significative. En plus d'autres administrations par voie orale, on peut favoriser des compositions à libération prolongée. D'autres voies d'administration peuvent comprendre, mais ne sont pas limitées, aux injections intraveineuses, intramusculaires et intrapéritonéales, aux implants sous-cutanés, aussi bien que les administrations buccales, sublinguales, transdermiques, topiques, rectales et intranasales.

Selon un mode particulier de réalisation, l'invention a pour objet l'utilisation d'un composé de formule (I) ou de ses sels pharmaceutiquement acceptables selon l'invention, caractérisée en ce que la dose journalière est de 20 à 60 mg chez l'adulte.

Le spécialiste pourra déterminer le dosage adéquat relatif à chaque patient ; ce dosage pourra varier en fonction de l'âge, du poids et de la réponse au traitement d'un patient donné. Les exemples de dosage ci-dessus sont représentatifs de la moyenne. Il est toutefois possible d'administrer des doses plus ou moins importantes par rapport à cette moyenne.

### Procédé de préparation des composés de formule (I) :

Selon l'invention, les composés tels que définis par la formule (I) peuvent être préparés par les procédés suivants à partir du traitement des composés optiquement actifs de formule (II) 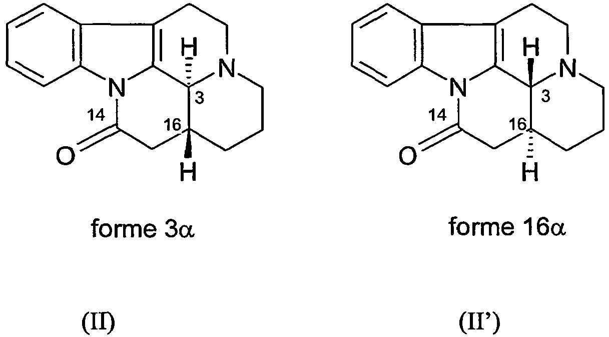 par un agent réducteur ; on obtient les deux couples de diastéréoisomères [(3α, 14α), (3α, 14β)] et [(14α, 16α), (14β, 16α)] de formule (I), ou leur mélange, et on traite, si désiré, le produit de la réaction par un acide minéral ou organique pour en former le sel.

Les produits de formule (II) et (II') peuvent être préparés par exemple comme indiqué dans la demande de brevet française publiée sous le numéro FR 2 190 113.

Le mélange racémique des composés de formule (II) peut être séparé par dédoublement.

Par réduction d'un des deux énantiomères de formule (II), on peut obtenir un couple de diastéréoisomères (±) de formule (I) ou des mélanges en proportions très variables des deux diastéréoisomères. L'expérience décrite dans la demande de brevet français publiée sous le numéro FR 2623503 montre que l'on n'obtient pratiquement qu'un seul des deux diastéréoisomères (voir exemple B).

Les composés de formule (II) utilisés peuvent l'être sous forme racémique ou optiquement active.

Le ou les composés de réduction de formule (I) obtenus à partir du produit de formule (II) le sont bien entendu sous la forme stéréochimique correspondante.

Les composés de formule (II) peuvent être utilisés sous forme de l'un de leurs sels d'addition avec les acides minéraux ou organiques. Si tel est le cas, on peut obtenir les produits de formule (I) sous forme salifiée ou non selon les conditions opératoires choisies.

Les mélanges racémiques ou optiquement actifs des composés de formule générale (I) peuvent également être préparés comme indiqué dans la demande de brevet française publiée sous le numéro FR 2 381 048 et dans la demande de certificat d'addition française publiée sous le numéro FR 2 433 528.

Dans des conditions préférentielles de mise en oeuvre de l'invention, le procédé ci-dessus décrit est réalisé de la manière suivante.

L'agent réducteur utilisé peut être un hydrure, notamment un hydrure mixte, tel que par exemple, l'hydrure mixte de lithium et d'aluminium, le diéthylhydrure de sodium et d'aluminium, l'hydroborure de sodium, l'hydroborure de lithium, l'hydrure de diisobutyl-aluminium.

La réaction de réduction est réalisée au sein d'un solvant organique ou d'un mélange de solvants tels que par exemple un éther comme l'éther éthylique, le tétrahydrofuranne, ou un hydrocarbure aromatique comme le toluène, le benzène, le xylène.

La réaction de réduction peut être réalisée à une température allant de - 20°C à la température de reflux du milieu réactionnel. Elle est avantageusement réalisée à température ambiante.

Dans le cas de l'utilisation comme agent réducteur d'un hydrure métallique, le composé de formule (I) est libéré du complexe formé intermédiairement avec l'hydrure, selon la pratique courante, par addition d'une solution aqueuse alcaline telle que par exemple une solution d'hydroxyde de sodium.

La réduction du composé (II) *trans* 3α peut conduire au composé (+) (3α, 14α) 14,15-dihydro 20,21-dinoréburnaménin 14-ol.

La réduction du composé (II') *trans* 16α peut conduire au composé (-) (14β, 16α) 14,15-dihydro 20,21-dinoréburnaménin 14-ol.

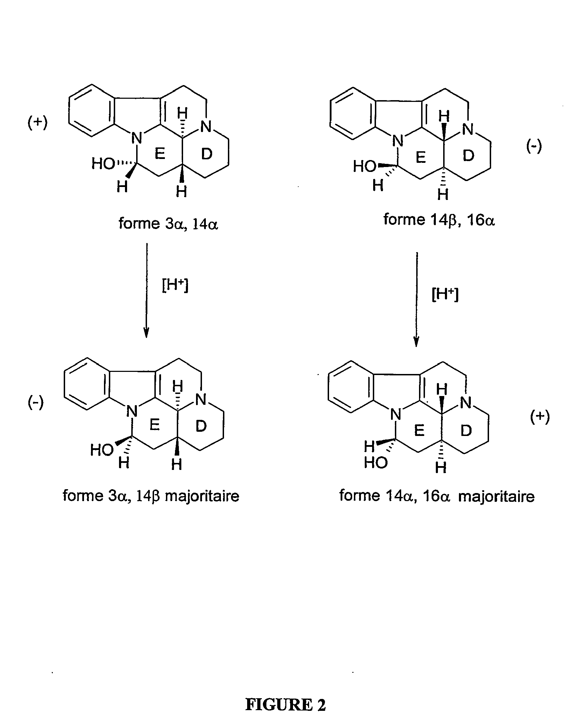

On peut traiter ces composés par un acide, par exemple de l'acide chlorhydrique pour obtenir respectivement les formes (-) (3α, 14β) 14,15-dihydro 20,21-dinoréburnaménin 14-ol et (+) (14α, 16α) 14,15-dihydro 20,21-dinoréburnaménin 14-ol majoritaires (cf. schéma ci-après et figure 2).

Schéma représentant la voie générale de synthèse des isomères optiquement actifs des composés de formule (I) à partir des composés de formule (ID (composés de formule (II) décrits dans le document de brevet belge publié sous le N° BE 764166)

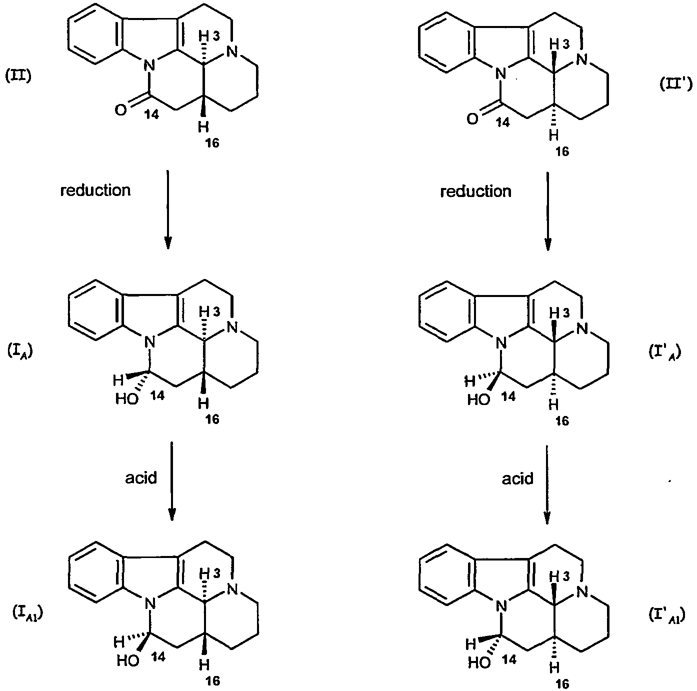

L'isolement de l'un ou de l'autre des disatéréoisomères de leur mélange peut être réalisé par des méthodes usuelles : chromatographie, cristallisation directe, solubilisation différentielle telle que par exemple la solubilisation différentielle dans le toluène à chaud.

Les légendes des figures et exemples qui suivent sont destinées à illustrer l'invention sans aucunement en limiter la portée.

### LEGENDES DES FIGURES

**Figure 1** **:** Représentation des quatre formes présentes dans le mélange racémique BC19 en solution acide
Comme indiqué, ces quatre formes correspondent à deux couples de diastéréoisomères qui présentent soit la configuration 16α soit la configuration 3α. Il n'y a pas de transformation spontanée possible de la configuration 3α (à gauche) vers la configuration 16α (à droite).

**Figure 2** **:** Représentation de la réaction permettant d'obtenir le diastéréoisomère (3α, 14β) à partir du (3α, 14α) et le diastéréoisomère (14α, 14α) à partir du (14β, 14α) sous l'action d'un acide.

### EXEMPLES

### Exemple 1 : Procédés de préparation des composés selon l'invention

### Exemple A : Procédés de préparation des mélanges racémiques tels que le BC19 ou optiquement actifs de l'invention

Les mélanges racémiques ou optiquement actifs des composés de formule générale (I) peuvent notamment être préparés comme indiqué dans la demande de brevet française publiée sous le numéro FR 2 381 048 et dans la demande de certificat d'addition française publiée sous le numéro FR 2 433 528.

### Exemple B : Procédés de préparation des diastéréoisomères de l'invention

Les diastéréoisomères (3α, 14α), (3α, 14β), (14α, 16α) et (14β, 16α) de formule (I) selon l'invention peuvent être obtenus comme décrit dans la demande de brevet français publiée sous le numéro FR 2623503. De tels procédés sont décrits brièvement ci-après.

### Exemple B1 : (14β, 16α) 14,15-dihydro 20,21-dinoréburnaménin-14-ol (I'_{A})

On dissout 10,8 g de (16α) (+) 20,21-dinoréburnaménin-14(15H) one dans 110 ml de toluène anhydre, ajoute en dix minutes, sous atmosphère inerte, 18,9 ml de dihydrure de diéthyl aluminium-sodium à 25 % dans le toluène et agite une heure à température ambiante. On hydrolyse par addition de 20 ml de soude 5 N et chauffe à 90°C pendant deux heures. On distille le toluène et simultanément, on introduit 100 ml d'eau. On amène à température ambiante, essore le produit obtenu, lave à l'eau, sèche sous pression réduite et récupère 10,7 g de produit attendu qui recristallise dans le méthanol et fond à 254 °C. [alpha]_{D} = -36 ° ± 1 ° (c = 0,6 % DMF).

Dichroïsme circulaire (dioxanne) :
Max. 225 nm Δε = - 8
Max. 237 nm Δε = + 9,5
Max. 280 nm Δε = -2

Spectre RMN ¹H (pyridine) 250 MHZ δ (ppm) : 5,78 (H porté par le C₁₄). Structure possible avec OH équatorial, OH axial non détecté. 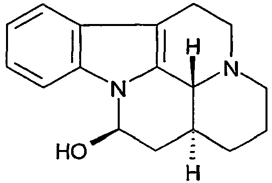 C₁₇H₂₀N₂O
M = 268,36 g/mol
F = 254°C

### Exemple B2 : (3α, 14α) 14,15-dihydro 20,21-dinoréburnaménin-14-ol (I_{A})

On opère comme à l'exemple 1 à partir de 15 g de (3α)(-)(20,21-dinoréburnaménin-14(15H)-one et obtient 15 g de produit attendu contenant très peu de produit ayant l'OH axial. Après recristallisation dans le méthanol, on obtient le produit fondant à 254°C.
[alpha]_{D} = + 32,5 ° ± 1 ° (c = 1 % DMF)
Dichroïsme circulaire (dioxanne) :
Max. 227 nm Δε = + 10
Max. 238 nm Δε = -10
Max. 288 nm Δε = + 2

Spectre RMN ¹H (pyridine) 250 MHZ δ (ppm) : 5,79 (H porté par le C₁₄). Structure possible avec OH équatorial, OH axial non détecté. 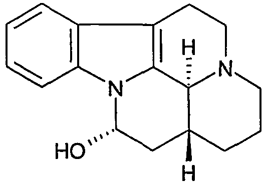 C₁₇H₂₀N₂O
M = 268,36 g/mol
F = 254 °C

### Exemple B3 : (14α, 16α) 14,15-dihydro 20,21-dinoréburnaménin-14-ol (I'_{A1})

On met en suspension 2,75 g du produit obtenu à l'exemple 1 dans 55 ml d'acide chlorhydrique 2 N et chauffe une heure trente minutes à 50 °C. On ajoute à la solution obtenue 55 ml d'eau glacée et amène à un pH alcalin par addition de 10 ml d'ammoniaque 22 Bé et agite 15 minutes à température ambiante. On essore le précipité, lave à l'eau, sèche à 50 °C sous pression réduite et obtient 2,75 g de produit (mélange OH axial et équatorial). On chromatographie ce dernier sous pression, sur silice, élue par un mélange acétate d'éthyle - méthanol - ammoniaque (97-3-0,3). On obtient 1,70 g de produit (OH axial). F = 234 °C.
[alpha]_{D} = + 150 ° ± 2 ° (c = 1 % DMF).
Dichroïsme circulaire (dioxanne) :
Max. : 230 nm Δε = + 19
Max. : 290 nm Δε= - 1,75
Spectre RMN ¹H (pyridine) 250 MHZ δ (ppm) : 6,26 (H porté par le C₁₄). Structure possible avec OH axial, OH équatorial non détecté. 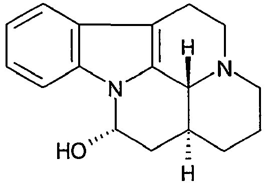
C₁₇H₂₀N₂O
M = 268,36 g/mol
F = 234°C

### Exemple B4 : (3α, 14β)14,15-dihydro 20,21-dinoréburnaménin-14-ol (I'_{A})

On opère comme à l'exemple 3 en partant de 13,3 g de produit obtenu à l'exemple 2 et obtient 7,7 g de produit (OH axial). F = 234°C
[alpha]_{D} = -152,5 ° ± 2,5 ° (c = 1 % DMF)
Dichroïsme circulaire (dioxanne) :
Max. : 228 nm Δε = - 20
Max. : 290 nm Δε = + 1,5
Spectre RMN ¹H (pyridine) 250 MHZ δ (ppm) : 6,23 (H porté par le C₁₄). Structure possible avec OH axial, OH équatorial non détecté. 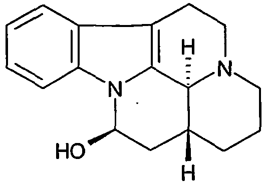
C₁₇H₂₀N₂O
M = 268,36 g/mol F = 234 °C

### Exemple 2 : Formes pharmaceutiques

a) comprimés : on a préparé des comprimés répondant à la formule suivante : 14,15-dihydro 20,21-dinoréburnaménin14-ol (BC19) : 30 mg
   Excipient q.s. pour un comprimé (détail de l'excipient : lactose, amidon de blé, amidon traité, amidon de riz, stéarate de magnésium, talc)
b) gélules : on a préparé des comprimés répondant à la formule suivante : 14,15-dihydro 20,21-dinoréburnaménin14-ol (BC19) : 30 mg
   Excipients : saccharose (115 mg/gél.), amidon, acide stéarique, lactose, talc, gomme laque, povidone, polymères méthacryliques.

### Exemple 3 : Etude pharmacologique - détermination de la toxicité aiguë du BC19 ou 14,15-dihydro 20,21-dinoréburnaménin14-ol

La toxicité aiguë est déterminée sur des lots de 10 souris mâles et femelles, d'un poids de 20-22 g, à jeun depuis la veille au soir.

Le produit est administré par voie intraveineuse, en solution dans du sérum physiologique, additionné de quelques gouttes d'acide chlorhydrique (les produits à tester sont alors en solution chlorhydrique).

La mortalité est relevée quotidiennement pendant une semaine. Les doses létales 50 (DL50) ont été déterminées par la méthode de Lichfield J.T. et Wilcoxon F. (J. Pharm. Exp. Therap.96:99, 1949). Les résultats obtenus n'ont mis en évidence aucune toxicité du composé BC19 aux doses efficaces.

### Exemple 4 (comparatif) : Application du BC19 ou 14,15-dihydro 20,21-dinoréburnaménin14-ol au traitement des désordres du cycle veille-sommeil

Les données anatomiques présentées dans le tableau 1 ci-après ont été obtenues trois jours après une injection unique ou après un traitement séquentiel de cinq injections à raison d'une injection tous les trois jours. Les cellules immunopositives pour la tyrosine hydroxylase (TH) et les fibres contenant de la noradrénaline ont été identifiées par immunocytochimie dans les aires de cerveau examinées. Les enregistrements du sommeil ont été réalisés sur un groupe de dix souris Balb/c. L'électroencéphalogramme de chaque animal a été enregistré en continu pendant cinq jours pour l'acquisition des données basales du cycle veille-sommeil. Cinq animaux ont ensuite été traités par cinq injections en IP tous les trois jours à partir du jour 5 jusqu'au jour 17 de l'expérience. Les cinq autres animaux ont reçu au même moment une injection de véhicule. A l'issue de la dernière injection toutes les souris ont été privées de sommeil pendant 6 heures et les enregistrements ont duré encore 2 jours consécutifs pour mesurer le rebond de sommeil REM total. Dans toutes ces expériences la dose de BC19 donnée est de 20 mg/kg pour chaque injection.

**Tableau 1 : Paramètres originaux modifiés par le traitement de souris Balb/c consanguines.**

| **Paramètres** | **Contrôles Balb/c** | **Balb/c traitées** | **traitement** |
|---|---|---|---|
| Nombre total de neurones immunopositifs pour la TH dans le LC | 910 ± 22 (100 ± 2%) | 1228 ± 24 (135 ± 3%)*** | sacrifice 3 jours après une unique injection en IP (20 mg/kg) |
| Nombre de neurones dans le tiers postérieur de groupe de cellules exprimant l'hypocrétine dans l'hypothalamus | 552 ± 40 (100 ± 7%) | 671 ± 24 (121 ± 4%)** | sacrifice 3 jours après une unique injection en IP (20 mg/kg) |
| Densité des fibres contenant de la noradrénaline (µm/µm²) dans le cortex préfrontal | 0,032 ± 0,002 (100 + 7%) | 0,050 ± 0,003 (156 ± 9%) | traitement séquentiel |
| Durée du sommeil REM durant la période de récupération (mn) | | | |
| sans privation de sommeil (contrôles) | 73 ± 10 (100 ± 13%) | | |
| durant la période de récupération (souris privées) | 74 ± 9 (101 ± 12 %) | 110 ± 8 151 ± 11*** | |

Ces résultats mettent en évidence que le composé BC19, lorsqu'il était injecté à des souris Balb/c permet :
- de restaurer le phénotype noradrénergique dans une population significative du locus caeruleus ;
- de restaurer l'innervation noradrénergique dans le cortex préfrontal
- de restaurer le phénotype hypocrétine dans une sous population de neurones de l'hypothalamus ; et
- inverser l'incapacité de ces souris de race consanguine à présenter un rebond du sommeil REM après privation de sommeil (REM pour « Rapid Eye Movement » le sommeil REM étant également appelé sommeil paradoxal).

Ainsi, ce composé apparaît actif dans le traitement des désordres du cycle veille-sommeil, incluant notamment la narcolepsie, l'hypersomnie, et l'état chronique d'hypoéveil.

## Revendications

1. Utilisation d'un composé général de formule (I) 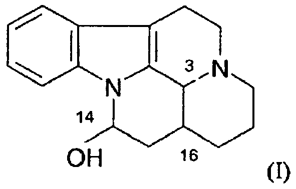 pour lequel l'atome d'hydrogène en position 3 et l'atome d'hydrogène en position 16 sont *trans,* le radical hydroxyle en position 14 pouvant être quant à lui sous la forme a ou β,
ou l'un de ses sels pharmaceutiquement acceptables, pour la préparation d'une composition pharmaceutique destinée à traiter et/ou à prévenir les dépressions majeures résistant aux traitements.

2. Utilisation d'un composé tel que décrit dans la revendication 1, pour la préparation d'une composition pharmaceutique destinée à rendre sensibles aux traitements antidépresseurs classiques des patients atteints de dépression majeure résistant à ces traitements antidépresseurs classiques.

3. Utilisation selon l'une des revendications 1 et 2, **caractérisée en ce que** le composé de formule (I) ou l'un de ses sels pharmaceutiquement acceptables est sous la forme d'un mélange racémique ou optiquement actif.

4. Utilisation selon l'une des revendications 1 à 3, **caractérisée en ce que** le composé de formule (I) ou l'un de ses sels pharmaceutiquement acceptables est choisi parmi les composés de formule (I) suivants :
a) (3α) (±) 14,15-dihydro 20,21-dinoréburnaménin 14-ol ; et
b) (16α) (±) 14,15-dihydro 20,21-dinoréburnaménin 14-ol, et dans lequel, le mélange des deux diastéréoisomères (+) et (-) présents dans ces composés a) et b) est en proportion équimolaire ou non.

5. Utilisation selon l'une des revendications 1 à 4, **caractérisée en ce que** le composé de formule (I) ou l'un de ses sels pharmaceutiquement acceptables est choisi parmi les composés de formule (I) suivants :
a) (3α, 14α) 14,15-dihydro 20,21-dinoréburnaménin 14-ol ;
b) (3α, 14β 14,15-dihydro 20,21-dinoréburnaménin 14-ol ;
c) (14α, 16α) 14,15-dihydro 20,21-dinoréburnaménin 14-ol ; et
d) (14β, 16α) 14,15-dihydro 20,21-dinoréburnaménin 14-ol.

6. Utilisation selon l'une des revendications 1 à 5, dans laquelle la composition pharmaceutique est administrée par voie orale, par voie intraveineuse, par voie intrapéritonéale ou par voie intramusculaire.

7. Utilisation selon l'une des revendications 1 à 6, selon laquelle on administre 20 à 60 mg du composé de formule (I) ou de l'un de ses sels pharmaceutiquement acceptables par jour chez le patient à traiter.

## Claims

1. Use of a compound with general formula (I) 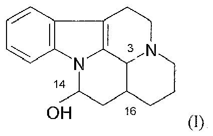 for which the hydrogen atom in position 3 and the hydrogen atom in position 16 are *trans,* the hydroxyl radical in position 14 possibly being in α or β form,
or any of the pharmaceutically acceptable salts thereof for the preparation of a pharmaceutical composition for the treatment or prevention of major depressions resistant to treatments.

2. Use of a compound as described in claim 1, for the preparation of a pharmaceutical composition to make patients suffering from major depression and resistant to classical antidepressant treatments, sensitive to these treatments.

3. Use according to any of claims 1 and 2, **characterised in that** the compound with formula (I) or any of the pharmaceutically acceptable salts thereof is in the form of a racemic or optically active mixture.

4. Use according to any of claims 1 to 3, **characterised in that** the compound with formula (I) or any of the pharmaceutically acceptable salts thereof is selected from among the following compounds with formula (I):
a) (3α) (±) 14,15-dihydro 20,21-dinoreburnamenin 14-ol; and
b) (16α) (±) 14,15-dihydro 20,21-dinoreburnamenium 14-ol,
and wherein the mixture of the two (+) and (-) diastereoisomers present in compounds a) and b) is in equimolar proportion or not.

5. Use according to any of claims 1 to 4, **characterised in that** the compound with formula (I) or any of the pharmaceutically acceptable salts thereof is selected among the following compounds with formula (I):
a) (3α, 14α) 14,15-dihydro 20,21-dinoreburnamenin 14-ol;
b) (3α, 14β) 14,15-dihydro 20,21-dinoreburnamenin 14-ol;
c) (14α, 16α) 14,15-dihydro 20,21-dinoreburnamenin 14-ol; and
d) (14β, 16α) 14,15-dihydro 20,21-dinoreburnamenin 14-ol.

6. Use according to any of claims 1 to 5, wherein the pharmaceutical composition is administered orally, intravenously, or by the intraperitoneal or intramuscular route.

7. Use according to any of claims 1 to 6, wherein 20 to 60 mg of the compound with formula (1) or any of the pharmaceutically acceptable salts thereof is administered daily to the patient to be treated.

## Patentansprüche

1. Verwendung einer allgemeinen Verbindung der Formel (1) 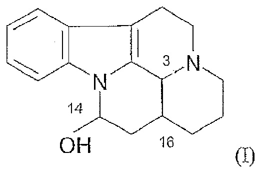 wobei das Wasserstoffatom in Position 3 und das Wasserstoffatom in Position 16 in trans-Konfiguration vorliegen und der Hydroxylrest in Position 14 in α- oder β-Form vorliegen kann,
oder eines ihrer pharmazeutisch verträglichen Salze, zur Herstellung eines Arzneimittels zur Behandlung und/oder Vorbeugung von behandlungsresistenten Major Depressionen.

2. Verwendung einer Verbindung wie in Anspruch 1 definiert zur Herstellung eines Arzneimittels zur Sensibilisierung von Patienten für Behandlungen mit klassischen Antidepressiva, wobei die Patienten an einer Major Depression leiden, die resistent gegenüber solchen Behandlungen mit klassischen Antidepressiva ist.

3. Verwendung gemäß einem der Ansprüche 1 und 2, **dadurch gekennzeichnet, dass** die Verbindung der Formel (1) oder eines ihrer pharmazeutisch verträglichen Salze in Form eines racemischen Gemisches oder in optisch aktiver Form vorliegt.

4. Verwendung gemäß einem der Ansprüche 1 bis 3, **dadurch gekennzeichnet, dass** die Verbindung der Formel (I) oder eines ihrer pharmazeutisch verträglichen Salze aus den folgenden Verbindungen der Formel (I) ausgewählt ist:
a) (3α) (±) 14,15 Dihydro-20,21 dinoreburnamenin-14-ol; und
b) (16α) (±) 74,15 Dihydro-20,21 dinoreburnamenin-14-ol,
und wobei in den Verbindungen a) und b) das Gemisch der zwei Diastereomere (+) und (-) in äquimolarem Verhältnis vorliegt oder nicht.

5. Verwendung gemäß einem der Ansprüche 1 bis 4, **dadurch gekennzeichnet, dass** die Verbindung der Formel (I) oder eines ihrer pharmazeutisch verträglichen Salze aus den folgenden Verbindungen der Formel (I) ausgewählt ist:
a) (3α, 14β) 14,15 Dihydro-20,21 dinoreburnamenin-14-ol;
b) (3α, 14β) 14,15 Dihydro-20,21 dinoreburnamenin-14-ol;
c) (14α, 16α) 14,15 Dihydro-20,21 dinoreburnamenin-14-ol; und
d) (14β, 16α) 14,15 Dihydro-20,21 dinoreburnamenin-14-ol.

6. Verwendung gemäß einem der Ansprüche 1 bis 5, wobei das Arzneimittel oral, intravenös, intraperitoneal oder intramuskulär verabreicht wird.

7. Verwendung gemäß einem der Ansprüche 1 bis 6, wobei dem zu behandelnden Patienten 20 bis 60 mg der Verbindung der Formel (1) oder eines ihrer pharmazeutisch verträglichen Salze pro Tag verahreicht werden.
